(19) Europäisches Patentamt
European Patent Office
Office européen des brevets

(11) Veröffentlichungsnummer: **0 025 887**
**B1**

# (12) EUROPÄISCHE PATENTSCHRIFT

(45) Veröffentlichungstag der Patentschrift: **21.09.83**

(51) Int. Cl.³: **C 07 C 147/107**

(21) Anmeldenummer: **80105027.9**

(22) Anmeldetag: **23.08.80**

(54) **Sulfonylcarbonsäurederivate.**

(30) Priorität: **04.09.79 DE 2935682**

(43) Veröffentlichungstag der Anmeldung: **01.04.81 Patentblatt 81/13**

(45) Bekanntmachung des Hinweises auf die Patenterteilung: **21.09.83 Patentblatt 83/38**

(84) Benannte Vertragsstaaten: **CH DE FR GB IT LI**

(56) Entgegenhaltungen:
**GB-A-1 452 174**
**US-A-3 140 306**
**US-A-3 225 085**

***Die Akte enthält technische Angaben, die nach dem Eingang der Anmeldung eingereicht wurden und die nicht in dieser Patentschrift enthalten sind.***

(73) Patentinhaber: **BAYER AG, Zentralbereich Patente, Marken und Lizenzen, D-5090 Leverkusen 1, Bayerwerk (DE)**

(72) Erfinder: **Schallner, Otto, Dr., Jakob-Böhme-Strasse 11, D-5000 Köln 80 (DE)**
Erfinder: **Oeckl, Siegfried, Dr., Andreas-Gryphius-Strasse 9, D-5000 Köln 80 (DE)**
Erfinder: **Schündehütte, Karl Heinz, Dr., Klief 75, D-5090 Leverkusen 3 (DE)**

**EP 0 025 887 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Sulfonylcarbonsäurederivate

Gegenstand der vorliegenden Erfindung sind die Säurehalogenide und Anhydride von Säuren der Formel

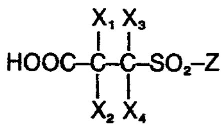

(I)

worin $X_1$ bis $X_4$ = Wasserstoff oder Halogen bedeuten, wobei nur einer oder zwei der Reste $X_1$ bis $X_4$ für Halogen stehen,

und Z = $C_1$–$C_4$-Alkyl, gegebenenfalls durch $C_1$–$C_4$-Alkyl oder Halogen substituiertes Phenyl bedeutet.

Bevorzugte Derivate von (I) sind dabei Säurehalogenide und Anhydride von Säuren der Formel (I)

worin a)

$X_1$ = Cl oder Br und $X_2$ bis $X_4$ = H

oder worin b)

$X_1$, $X_2$, $X_4$ = H und $X_3$ = Cl oder Br.

Die Herstellung der neuen Derivate von (I) erfolgt in an sich bekannter Weise durch Umsetzung der bekannten Säuren (I) mit organischen oder anorganischen Säurehalogeniden oder Säureanhydriden bei etwa 0–150 °C, bevorzugt 10–120 °C.

Geeignete Säurehalogenide sind beispielsweise Sulfurylchlorid, Thionylhalogenide, wie Thionylchlorid, Phosphorhalogenide wie Phosphortribromid und Phosphoroxychlorid sowie Phosgen, weiterhin Carbonsäurechloride wie Benzoylchlorid, Sulfonsäurechloride wie Toluolsulfochlorid und halogenierte Heterocyclen wie Trifluortriazin.

Geeignete Säureanhydride sind beispielsweise die aliphatischen oder aromatischen Mono- oder Dicarbonsäureanhydride, wie Acetanhydrid, Phthalsäureanhydrid sowie Anhydride anorganischer Säuren wie Phosphorpentoxid.

Im allgemeinen arbeitet man dabei mit stöchiometrischen Mengen, gebenenfalls in Gegenwart üblicher inerter Lösungsmittel. Es können aber auch die Reaktanten als Lösungsmittel verwendet werden.

Die Umsetzung kann auch in Gegenwart von Halogenwasserstoffakzeptoren erfolgen, bevorzugt in Mengen von 0,01–0,9 Mol je Mol (I).

Die neuen Verbindungen sind Zwischenprodukte, beispielsweise für die Herstellung neuer Reaktivfarbstoffe, die man durch Umsetzung mit entsprechenden aminogruppenhaltigen Farbstoffen in üblicher Weise erhält.

Aus GB-A 973 786 sind Farbstoffe bekannt, welche die

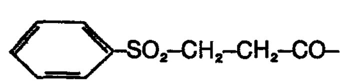

Gruppe enthalten.

Weiterhin sind aus The Chemistry of Synthetic Dyes (Venkataraman) Volume VI, S. 11, Academic Press New York und London (1972) Farbstoffe bekannt, die die

$H_3C$–$SO_2$–$CH_2$–$CH_2$–CO-Gruppierung enthalten.

Die den Farbstoffen aus den beiden vorgenannten Publikationen entsprechenden und aus erfindungsgemässen Säurehalogeniden und Säureanhydriden erhaltenen Farbstoffe zeigen demgegenüber überraschende färberische Eigenschaften.

## Beispiel 1

248,5 g (1 Mol) trockene 2-Chlor-3-phenylsulfonylpropionsäure werden bei 20 °C in 300 ml Thionylchlorid suspendiert. Man erwärmt die Suspension langsam auf 60 °C. Nach etwa 7 Stunden ist die Gasentwicklung beendet. Aus der entstandenen klaren Lösung wird das überschüssige Thionylchlorid unter vermindertem Druck bei 240–60 °C abdestilliert. Der Rückstand besteht aus 2-Chlor-3-phenylsulfonylpropionylchlorid, das beim Erkalten auskristallisiert. Die Ausbeute ist quantitativ. Es hat folgendes $^1$H–NMR-Spektrum; gemessen in Deuterochloroform: δ = 3,83 (Multiplett, 2 H), 4,93 (doppeltes Dublett, 1 H), 7,50–8,10 (Multiplett, 5 H). Die aus Toluol umkristallisierte Verbindung hat einen Schmelzpunkt von 49 °C.

## Beispiel 2

293 g (1 Mol) trockene 2-Brom-3-phenylsulfonylpropionsäure werden bei 20 °C in 350 ml Thionylchlorid suspendiert. Man versetzt mit 2 ml Dimethylformamid und erwärmt die Suspension langsam auf 60 °C. Nach etwa 2 Stunden ist die Gasentwicklung beendet. Aus der entstandenen klaren Lösung wird das überschüssige Thionylchlorid unter reduziertem Druck bei 40–60 °C abdestilliert. Der Rückstand besteht aus 2-Brom-3-phenylsulfonylpropionylchlorid, das beim Erkalten auskristallisiert. Die Ausbeute ist quantitativ. Es hat folgendes $^1$H–NMR-Spektrum, gemessen in Deuterochloroform: δ = 3,68 (doppeltes Dublett, 1 H), 4,13 (Triplett, 1 H), 4,92 (doppeltes Dublett, 1 H), 7,50–8,10 (Multiplett, 5 H).

Die aus Toluol umkristallisierte Verbindung hat einen Schmelzpunkt von 47 °C.

## Beispiel 3

305 g (1 Mol) feuchte 3-Chlor-3-phenylsulfonylpropionsäure werden in 350 ml Chlorbenzol suspendiert. Man entwässert azeotrop, kühlt die Mischung auf 20–30 °C ab, gibt 150 g Thionylchlorid zu und erwärmt langsam auf 60 °C. Nach beendeter Gasentwicklung wird das überschüssige Thionylchlorid und das Lösungsmittel unter vermindertem Druck bei 40–60 °C abdestilliert. Der Rückstand besteht aus 3-Chlor-3-phenylsulfonylpropionylchlorid, das schon teilweise bei der Destillation auskristallisiert. Die Ausbeute ist quantitativ.

Es hat folgendes $^1$H–NMR-Spektrum, gemessen in Deuterochloroform: δ = 3,42 (Multiplett, 1 H),

4,08 (doppeltes Dublett, 1 H), 5,12 (doppeltes Dublett, 1 H), 7,40–8,20 (Multiplett, 5 H).

Die aus Chlorbenzol umkristallisierte Verbindung hat einen Schmelzpunkt von 99 °C.

Verwendet man statt des Chlor-Derivates die 3-Brom-3-phenylsulfonylpropionsäure und verfährt wie oben angegeben, so erhält man in ebenfalls quantitativer Ausbeute 3-Brom-3-phenylsulfonylpropionylchlorid $^1$H–NMR ($DCCl_3$): δ = 3,50 (doppeltes Dublett, 1 H), 4,17 (doppeltes Dublett, 1 H), 5,16 (doppeltes Dublett, 1 H), 7,40–8,15 (Multiplett, 5 H). Schmelzpunkt, aus Toluol umkristallisiert: 96 °C

Beispiel 4

283 g (1 Mol) trockene 2,2-Dichlor-3-phenylsulfonylpropionsäure werden bei 20 °C in 400 ml Thionylchlorid suspendiert. Man versetzt mit 2 ml Dimethylformamid und erwärmt langsam auf 70–75 °C. Nach 4 Stunden ist die Gasentwicklung beendet. Aus der entstandenen klaren Lösung wird das überschüssige Thionylchlorid unter vermindertem Druck bei 40–60 °C abdestilliert. Der Rückstand besteht aus 2,2-Dichlor-3-phenylsulfonylpropionylchlorid. Die Ausbeute ist quantitativ.

Das Säurechlorid zeigt folgendes $^1$H-NMR-Spektrum ($CCl_4$): δ = 4,38 (Singulett, 2 H), 7,40–8,10 (Multiplett, 5 H).

Die aus Toluol umkristallisierte Verbindung hat einen Schmelzpunkt von 57 °C.

**Patentansprüche**

1. Säurehalogenide und Anhydride von Säuren der Formel

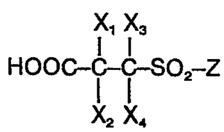

worin $X_1$ bis $X_4$ = Wasserstoff oder Halogen bedeuten, wobei nur einer oder zwei der Reste $X_1$ bis $X_4$ für Halogen stehen, und

Z = $C_1$–$C_4$-Alkyl, gegebenenfalls durch $C_1$–$C_4$-Alkyl oder Halogen substituiertes Phenyl bedeutet.

2. Säurehalogenide und Anhydride von Säuren der Formel gemäss Anspruch 1, worin $X_1$ = Cl oder Br und $X_2$ bis $X_4$ = H bedeuten.

3. Säurehalogenide und Anhydride von Säuren der Formel gemäss Anspruch 1, worin $X_1$, $X_2$, $X_4$ = H und $X_3$ = Cl oder Br bedeuten.

**Claims**

1. Acid halides and anhydrides of acids of the formula

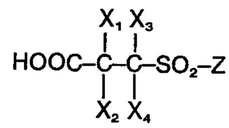

wherein

$X_1$ to $X_4$ = hydrogen or halogen, only one or two of the radicals $X_1$ to $X_4$ representing halogen, and

Z = $C_1$–$C_4$-alkyl, phenyl which is optionally substituted by $C_1$–$C_4$-alkyl or halogen.

2. Acid halides and anhydrides of acids of the formula according to claim 1, wherein $X_1$ = Cl or Br and $X_2$ to $X_4$ = H.

3. Acid halides and anhydrides of acids of the formula according to claim 1, wherein $X_1$, $X_2$ and $X_4$ = H and $X_3$ = Cl or Br.

**Revendications**

1. Halogénures et anhydrides d'acides de formule

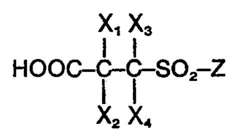

dans laquelle $X_1$ à $X_4$ désignent l'hydrogène ou un halogène, un ou deux seulement des restes $X_1$ à $X_4$ représentant un halogène, et

Z désigne un groupe alkyle en $C_1$ à $C_4$, ou phényle éventuellement substitué par un radical alkyle en $C_1$ à $C_4$ ou un halogène.

2. Halogénures et anhydrides d'acides de formule suivant la revendication 1, où $X_1$ désigne Cl ou Br et $X_2$ à $X_4$ désignent H.

3. Halogénures et anhydrides d'acides de formule suivant la revendication 1, où $X_1$, $X_2$, $X_4$ représentent H et $X_3$ représente Cl ou Br.